# EUROPEAN PATENT SPECIFICATION

(11) **EP 3 877 023 B1**
(45) Date of publication and mention of the grant of the patent: **31.07.2024**
(21) Application number: 19797299.5
(22) Date of filing: 07.11.2019
(51) Int. Cl.: A61M 15/00, B05B 11/00

(54) **RESERVOIR FOR AN INHALATION DEVICE**
RESERVOIR FÜR EINE INHALATIONSVORRICHTUNG
RÉSERVOIR POUR DISPOSITIF D'INHALATION

(30) Priority: 09.11.2018 US 201862757965 P; 09.11.2018 EP 18205390
(43) Date of publication of application: 15.09.2021
(73) Proprietor: invoX Belgium NV, 3590 Diepenbeek (BE)
(72) Inventor: RAWERT, Jürgen, 50933 Köln (DE)
(74) Representative: Synergy IP Group AG
(86) International application number: PCT/EP2019/080480
(87) International publication number: WO 2020/094761

(56) References cited:
- WO-A1-2015/091539
- WO-A1-2016/012102
- WO-A2-2010/133294
- US-B2- 9 265 910

## Description

### Field of the invention

The invention relates to the field of inhalation devices for medically active liquids. In particular, the invention relates to a cartridge containing said liquid for such inhalation devices.

### Background of the Invention

Nebulizers or other aerosol generators for liquids are known from the art since a long time ago. Amongst others, such devices are used in medical science and therapy. There, they serve as inhalation devices for the application of active ingredients in the form of aerosols, i.e. small liquid droplets embedded in a gas. Such an inhalation device is known e.g. from document EP 0 627 230 B1. Typical components of this inhalation device are: a housing which can be held in the hand of the operator; a reservoir in which the liquid that is to be aerosolized is contained, and a holder for holding said cartridge; downstream this reservoir a pumping unit for generation of a pressure being sufficiently high for nebulizing; connected to this pumping unit a means for the storage of potential energy such as a spring; and downstream this pumping unit an atomizing device in the form of one or several nozzles.

By means of the pumping unit, the liquid is drawn in a discrete amount, i.e. not continuously, from the reservoir, and fed to the nozzle. The pumping unit works without propellant and generates pressure preferably mechanically, by being driven by aforesaid spring.

An improvement of such an inhalation device is disclosed in patent application PCT/EP2018/061056, filed by the same applicant as the present invention.

This inhalation device makes use of a pumping unit with a pumping chamber formed by a hollow cylinder into which a moveable and hollow piston can be inserted in order to decrease the interior volume of said chamber, thus increasing the pressure both in said chamber and the inside of the piston, eventually leading to atomization of the liquid from the nozzle. By subsequently extracting the piston from the chamber, its interior volume is increased, and the resulting pressure decrease leads to drawing liquid from the reservoir into the chamber, such that a new atomizing cycle can begin.

In order to protect the reservoir which is often designed as a collapsible bag from damage, and to facilitate exchanging an empty reservoir for a fresh one, the reservoir is incorporated into a rigid cartridge. The cartridge can be inserted into, and extracted from, the interior of the housing, which provides an according fluidic-mechanical interface to mechanically fix the cartridge, and to connect it to the downstream arranged components.

In order for the collapsible bag to function properly, the pressure inside and outside the bag must be substantially identical. When liquid is drawn from the bag in order to fill the pumping chamber, the volume of the bag is accordingly reduced. To avoid building up an increasingly strong underpressure inside the cartridge, the latter must allow to compensate the aforesaid volume reduction. This is achieved by provision of at least one ventilation bore that allows ambient air to flow into the cartridge when the bag shrinks.

On the other hand, after fabrication and filling, quite a long time can pass prior to first use of the cartridge. In fact, the shelf life typically amounts to a year or even more. During this period, the aforesaid ventilation bore results in an exchange of gas between the inside and the outside of the cartridge. Said exchange is undesired, since liquid components which possibly evaporate through the bag can reach the outside, and incoming air increases this process of unwanted evaporation.

In document DE 199 40 713 A1, a "cartridge for a liquid" is disclosed which avoids these contravening requirements by providing a cartridge with a membrane that initially closes a ventilation bore located at the bottom of the cartridge. Upon insertion of the cartridge into the housing, the membrane is pierced by a needle and thus, permanently opened. Once broached, the cartridge must be used up within a certain period of time.

WO2016/012102 describes a delivery device having a replaceable cartridge.

A drawback of this solution is that said period of time can be rather short. Therefore, if the device is not used for a longer time, the cartridge must be disposed of and replaced, even when the bag is not yet emptied.

### Summary of the Invention

In a first aspect, the present invention provides a cartridge (1) for the storage of a medically active liquid (L) and for supply of the same to a pumping unit of an inhalation device,
- said inhalation device comprising a housing, a pumping unit which can be fluidically connected with said cartridge (1), a holder (2) for said cartridge (1), and a nozzle which is fluidically connected downstream of said pumping unit,
- wherein, when the pumping unit is fluidically connected to the cartridge (1), the pumping unit is capable of generating a negative gauge pressure such as to draw medically active liquid (L) from the cartridge (1) into the pumping unit, and of generating a positive gauge pressure such as to press said liquid (L) from said pumping unit into and through the nozzle such as to atomize said liquid (L),
- wherein the cartridge (1) comprises an outer container (3) enclosing a collapsible inner bag (4), and a cap (5) which is capable of closing the container (3), the cap (5) further providing an openable access opening (6) to the inner bag (4),
- wherein further, the container (3) of the cartridge (1) has at least one pressure compensation opening (7),
and wherein said pressure compensation opening (7) is located in a region (8) of the container (3) which is covered by the cap (5).

In a second aspect, the invention provides a holder (2) for the cartridge (1) of the first aspect of the invention, wherein the holder (2) is adapted to releasably connect to the cartridge (1) by clamping its cap (5).

In a third aspect, the invention provides an inhalation device for delivering a medically active liquid in the form of an aerosol, said inhalation device comprising a cartridge (1) according to the first aspect of the invention and a holder (2) according to the second aspect of the invention for said cartridge.

Further described is a method for the treatment, stabilization or prevention of a pulmonary disease or condition by inhalative administration of a medically active liquid in aerosolized form to patient in need thereof, wherein the medically active liquid in aerosolized form is generated and administered to said patient by an inhalation device according to the third aspect of the invention, as well as for a method for providing a pressure compensation to the collapsible inner bag-containing interior of the outer container (3) of a cartridge (1) according to the first aspect of the invention.

### Object of the Invention

The object of the invention is the provision of a cartridge for an inhalation device that avoids the drawbacks of the known art. In particular, a cartridge according to the invention should allow for a longer storage time of an already broached cartridge when not in use.

### Detailed Description of the Invention

The object is solved by a cartridge for the storage of a medically active liquid and for supply of the same to a pumping unit of an inhalation device,
- said inhalation device comprising a housing, a pumping unit which can be fluidically connected with said cartridge, a holder for said cartridge, and a nozzle which is fluidically connected downstream of said pumping unit,
- wherein, when the pumping unit is fluidically connected to the cartridge, the pumping unit is capable of generating a negative gauge pressure such as to draw medically active liquid from the cartridge into the pumping unit, and of generating a positive gauge pressure such as to press said liquid from said pumping unit into and through the nozzle such as to atomize said liquid,
- wherein the cartridge comprises an outer container enclosing a collapsible inner bag, and a cap which is capable of closing the container, the cap further providing an openable access opening to the inner bag,
- wherein further, the container of the cartridge has at least one pressure compensation opening,
and wherein said pressure compensation opening is located in a region of the container which is covered by the cap.

The object is, furthermore, solved by the inhalation device comprising the abovementioned cartridge and a holder for said cartridge, wherein the holder is adapted to releasably connect to the cartridge by clamping its cap.

Specific and/or advantageous embodiments of the aspects of the present invention are described in the respective dependent claims, the subsequent description, as well as the accompanying figures.

Unless the context clearly indicates or requires otherwise, the terms 'comprise', 'comprises' and 'comprising' and similar expressions are to be construed in an open and inclusive sense, as 'including, but not limited to' in this description as well as in the claims.

The cartridge according to the first aspect of the invention serves for the storage of a medically active liquid and for supply of the same to a pumping unit of an inhalation device as described above, i.e. an inhalation device comprising a housing, a pumping unit which can be, or is, fluidically connected with said cartridge, a holder for holding said cartridge inside the housing, and at least one nozzle which is fluidically connected (with the pumping unit) downstream of said pumping unit.

Said pumping unit is, or can be, fluidically connected to the cartridge, and the pumping unit is capable of generating a negative gauge pressure such as to draw medically active liquid from the cartridge into the pumping unit, and of generating a positive gauge pressure such as to press said liquid from said pumping unit into and through the at least one nozzle such as to atomize (or nebulize) said liquid.

The cartridge comprises an outer container enclosing a collapsible inner bag, and a cap which is capable of closing the container. In specific embodiments, the cap is capable of airtight closing the container. The cap further provides an openable access opening to the inner bag, i.e. an opening which allows for a fluid connection or coupling of the inside of the bag and the pumping unit, or more precisely, the pumping chamber of the pumping unit. Typically, said cap and access opening are located at the downstream end of the cartridge, typically referred to as the "top" of the cartridge and typically located opposite of the upstream end or "bottom" of the cartridge. However, it is clear that all spatial indications are not meant in a limiting way, since the inhalation device can be used in any orientation. However, for the sake of clarity, in the following, it is assumed that the cartridge is inserted into the bottom of the upright housing of the inhalation device, and that the at least one nozzle is located in the device's top region.

Further, the container of the cartridge has at least one pressure compensation opening, functioning as the aforesaid ventilation bore and connecting the space between the outer container and the collapsible inner bag to ensure that the pressure within that space corresponds to the pressure of the surrounding atmosphere in case the collapsible inner bag does not completely fill the inner lumen of the outer container of the cartridge. The term 'at least one pressure compensation opening' as used herein means that the cartridge has just one pressure compensation opening or a plurality of pressure compensation openings, such as 2 to about 10, or 2 to 8, or 2 to 6 or 2 to 5, or 2 to 4 or 2 or 3 pressure compensation openings. In specific embodiments, the cartridge has just one pressure compensation opening. In further specific embodiments, the cartridge has 2 pressure compensation openings.

In case of the presence of a plurality of pressure compensation openings, such as 2 or 3 or 4 or 5, or 2 or 3 or just 2 pressure compensation openings, in specific embodiments, these pressure compensation openings may be located on opposite or evenly spaced positions of the cartridge to ensure uniform pressure compensation within the interior of the cartridge. For example, the pressure compensation openings may be located along a perimeter or circumference around the main symmetry axis of the cartridge, preferably in a spaced or evenly spaced manner (such symmetry axis connecting the top and the bottom of the cartridge).

Accordingly, by means of this opening, the interior pressure of the cartridge can be adjusted to the ambient pressure by letting air flow into, or out of, the cartridge.

According to the invention, said pressure compensation opening is located in a region of the container which is covered by the cap. In other words, said pressure compensation opening is located within an area or surface area of the container, preferably at the top or downstream end of the container that is covered by the cap when the cartridge in disconnected from the holder or inhaler, respectively.

In the case of a plurality of pressure compensation openings as described above are provided in the container, preferably all of said openings are located within an area or surface area of the container, preferably at the top or downstream end of the container that is covered by the cap when the cartridge in disconnected from the holder or inhaler, respectively.

More specifically, said area or region of the container is covered by a surface of the cap which faces the container when disconnected from the holder or inhalation device as described in further detail below. Preferably, said cover is provided in an airtight manner.

In other words, while, according to the state of the art, the pressure compensation opening is located at the bottom of the cartridge and thus, spaced apart from said cap, in contrast, according to the invention, the pressure compensation opening is not only located close to the cap, and in top region of the cartridge, but in a way that is in fact covered by said cap.

The location of the opening under the cap has an important advantage which will become apparent from the subsequent description.

According to the invention, the cartridge can be separated from the rest of the device, or it can be installed inside the device. Thus, two "states" exist.

In a state in which the cartridge is disconnected from the holder, the pressure compensation opening is covered by the cap, preferably in an airtight manner. In a state when the cartridge is connected to the holder, said pressure compensation opening is open to allow for the pressure compensation or equilibration between the inner lumen of the cartridge and the surrounding atmosphere, while the pressure compensation opening as well as the surrounding area may still be shielded by the cap to ensure protection of the pressure compensation opening and the surrounding area from external contamination.

This means that preferably no gas exchange between the inside of the cartridge and its external environment or other form of contamination of the inner lumen of the cartridge by external contaminants is possible when the cartridge is separated from the device, and in particular, from the holder. In addition to this, even an already broached cartridge remains intact for a longer time, since liquid which possibly evaporates from the bag cannot leave the interior of the cartridge. This prevents or reduces a loss of liquid, while at the same time protecting the environment from possibly harmful substances emanating from the shelved device.

Only upon insertion of the cartridge into the specifically designed corresponding holder, the pressure compensation opening is opened, which is necessary for proper functioning of the device by allowing pressure compensation between the surrounding atmosphere and the inner lumen of the cartridge.

It is clear that the cartridge must be removed from the device, or at least from the holder, when the device is not used for a longer time in order to achieve the desired closing of the pressure compensation opening.

In specific embodiments, the housing is designed such that, even when the opening is closed, e.g. by spacing it apart from the holder, the cartridge can remain inside the housing. Preferably, the state of connection or disconnection is detectable from the outside, i.e. without opening the housing. This can be achieved by a simple window, or an at least partially transparent housing that allows detection of the position of the cartridge with respect to the holder.

In further specific embodiments, the cap and/or at least a cap connection region or area of the outer container are made of a deformable material. Further, the cap or at least said region can be deformed such that an air gap is provided between the surface of the cap which faces the container and the surface of the outer container which faces the cap to allow for air flow and pressure compensation between the inner lumen or interior of the cartridge and the surrounding atmosphere by gas exchange via the pressure compensation opening.

In other words, by exerting mechanical forces to, and thus, deforming, the cap or said region, the region which initially (in the detached or disconnected state) closes the aforesaid opening, lifts from said opening and provides said air gap. It is clear that the gap must directly or indirectly provide access to the outside. In other words, the air gap allows gas exchange between the interior of the cartridge and its exterior environment. An indirect access would e.g. lift the cap only such that a bypass is provided to e.g. a permanently present channel that itself leads to the outside. A direct access is provided when no such additional channel or the like is necessary.

The expression "interior" as used herein in this context refers to the space between the outside walls of the bag and the inside walls of the container.

In specific embodiments, the deformable material is elastically deformable. Thus, after stopping aforesaid exertion of forces, the initial shape of the cap is automatically retrieved or restored. However, further embodiments with a plastically deformable material are possible as well; in these cases, actively counteracting forces might be required in order to obtain the original shape of the cap.

In preferred embodiments, the cap is deformable by a radial compression force. This means that, for example, by providing force vectors which are circumferentially arranged around the cap, said cap can be deformed according to the invention. Besides a substantially uniform distribution of a "ring" of forces, according to one embodiment, a set of one, two, three or four vectors is usually sufficient to result in the desired deformation.

According to further embodiments, the cap has at its outer circumference at least one protrusion, which, in the connected state of the cartridge, causes the cap to be deformed such as to provide said air gap. In other words, in these embodiments the holder is designed such that, when the cartridge is inserted, the inside wall of the holder presses against said at least one protrusion. The force that forms due to said mechanical contact results in the aforesaid deformation.

It is clear that, instead of (or additionally to) said outer protrusion(s) of the cap, the outside of the cap can be designed plane, and inner protrusion(s) are provided at an inside wall of the holder which faces the cap.

It is further clear for the one skilled in the art how to design one or more protrusion(s) in order to obtain the desired one or more forces along aforesaid vector(s). It is also clear that, instead of a single or multiple protrusions, a circumferential protruding ring can be used, if a circumferentially acting force is desired. In one embodiment, the protrusion(s) or ring, respectively, serve also as a mechanical clip which is used for detachably fixing the cartridge inside the holder in a pre-determined connecting position.

As mentioned before, in preferred embodiments, the cap and/or the holder have a plurality of evenly spaced protrusions.

In a second aspect, the invention also relates to a holder for a cartridge as defined above in connection with the first aspect of the invention. According to this second aspect of the invention, the holder is adapted to releasably connect to the cartridge by clamping its cap, i.e. by clamping the cap of the cartridge. In other words, the holder provides the possibility to serve as a temporary fixation for the cap, and thus, for the entire cartridge, within the housing of the inhalation device. Such a holder may have further features. However, the holder must at least be suitable to mechanically hold the cartridge in place.

Depending on the specific construction of the cap, preferably, the holder enables interaction with the aforesaid protrusion(s) in order to provide said deforming forces.

According to another embodiment, the holder itself (instead of the cap, or in addition to the cap) is at least partially made from an elastic material and deforms upon insertion of the cartridge, thus capable of providing said air gap as described above. The mechanism as well as the aforementioned embodiments regarding the protrusion(s) etc. can be applied to or transferred into the holder without problems by the one skilled in the art.

Further, according to the invention, by way of a connection of the holder with a cartridge as described above, the air gap can be provided. In particular, the holder must, upon insertion of the cartridge, allow a formation of mechanical forces which result in a mechanical deformation of the cap, which, in turn, is needed to provide the aforementioned air gap.

In one embodiment, when the cap is connected to the holder, the holder is configured to exert a compression force (or compression forces) onto the cap.

In another embodiment, said forces can be tensile forces. Such forces, as well, can be used for generating or "opening" the aforesaid air gap, depending on the specific construction. However, compression forces are advantageous since they are easier to generate, and can, at the same time, be used for clamping the cap.

In a third aspect, the present invention provides an inhalation device for delivering a medically active liquid in the form of an aerosol, said inhalation device being adapted for holding or receiving a cartridge according the first aspect of the invention and comprising a holder according to the second aspect of the invention for holding said cartridge. Furthermore, according to this aspect, the present invention relates to an inhalation device for delivering a medically active liquid in the form of an aerosol, said inhalation device comprising a cartridge according to the first aspect of the invention and a holder according to the second aspect of the invention for holding said cartridge.

It should be noted that with regard to this aspect as well as the following aspects also, all embodiments, preferred embodiments and combinations thereof as described above in connection with the first and/or the second aspect of the invention apply correspondingly.

The term 'medically active liquid' as used herein is to be understood in a broad sense and, in specific embodiments, means a liquid or liquid composition that may be useful for the treatment, stabilization or prevention of a condition, disorder or disease, specifically of a pulmonary condition, disorder or disease of an animal or human, preferably of a human, that may be contained in the cartridge according to the first aspect of the invention and, accordingly, may be administered by the inhalation device according to the third aspect of the invention.

In specific embodiments, a 'medically active liquid' may be a compound or a mixture of compounds *per se.* In other specific embodiments a medically active liquid may be a solution, suspension or dispersion of an ingredient or active ingredient in a physiologically acceptable carrier or liquid. In further specific embodiments, the physiologically acceptable carrier liquid may be water or an aqueous mixture comprising water and one or more further physiologically acceptable solvents such as ethanol, propylene glycol or polyethylene glycol.

In specific embodiments, the term 'medically active liquid' as used herein may refer to a medically active liquid in form of a pharmaceutical composition comprising at least one active pharmaceutical ingredient (API), more specifically at least one inhalable active pharmaceutical ingredient. More specifically, such at least one inhalable active pharmaceutical ingredient may, for example, be selected from long-acting muscarinic antagonists (LAMA), long-acting beta agonists (LABA) and inhalable glucocorticoids (ICS), as well as from analgetics and antidiabetics, either alone or in combination which each other.

Examples for long-acting muscarinic antagonists (LAMA) that may be comprised by the medically active liquid as referred to herein comprise, but are not limited to aclidinium bromide, glycopyrronium salts, such as glycopyrronium bromide, revefenacin, tiotropium, such as tiotropium bromide, umeclidinium bromide, oxitropium bromide, flutropium bromide, ipratropium bromide, trospium chloride, tolterodine.

Examples for long-acting beta agonists (LABA) that may be comprised by the medically active liquid as referred to herein comprise, but are not limited to, albuterol, arformoterol, bambuterol, bitolterol, broxaterol, carbuterol, clenbuterol, fenoterol, formoterol, hexo-prenaline, ibuterol, indacaterol, indacterol, isoetharine, isoprenaline levosalbutamol, mabuterol meluadrine, metaproterenol, olodaterol, orciprenaline, pirbuterol, procaterol, reproterol, rimiterol, ritodrine, salmeterol, salmefamol, soterenot, sulphonterol, tiaramde, terbutaline, terbuterol.

Examples of inhalable glucocorticoids (ICS) that may be comprised by the medically active liquid as referred to herein comprise, but are not limited to, prednisolone, prednisone, butixocort propionate, flunisolide, beclomethasone, triamcinolone, budesonide, fluticasone, mometasone, ciclesonide, rofleponide, dexamethasone, etiprednol-dichloro-acetat, deflazacort, etiprednol, loteprednol, RPR-106541, NS-126, ST-26.

Furthermore, active pharmaceutical ingredients that may be comprised by the medically active liquid as referred to herein may be selected from analgetics, such as opioid analgetics (e.g. morphine, fentanyl) or non-opioid analgetics (e.g. salicylic acid derivates, e.g. acetylsalicylic acid) or cannabinoids (e.g. tetrahydrocannabinol), antidiabetics, such as insulin.

The medically active liquid or liquid pharmaceutical composition that may be administered in nebulized or aerosolized form by the present inhalation device may comprise at least one active pharmaceutically ingredient as described above, but may also comprise a mixture of two or more active pharmaceutically ingredients that may be administered by inhalation.

The medically active liquid or pharmaceutical composition that may be administered in nebulized or aerosolized form by the inhalation device according to the invention and, accordingly may be contained in the cartridge according to the first aspect of the invention is preferably formulated as a composition that is suitable, and adapted for inhalative use, in other words a composition that may be nebulized or aerosolized for inhalation and that is physiologically acceptable for inhalation by a subject.

The medically active liquid or pharmaceutical composition that may be administered by the inhalation device according to this aspect of the invention or contained in the cartridge of the invention may be in the form of a dispersion, for example a suspension with a liquid continuous phase, and a solid dispersed phase or in the form of a solution.

In further embodiments, the medically active liquid or pharmaceutical composition as described above may comprise, optionally and in addition to the one or more active pharmaceutical ingredient, one or more physiologically acceptable excipients, which are suitable for inhalative use. Excipients which may be featured in the composition may include, but are not limited to, one or more buffering agents to regulate or control the pH-value of the solution, salts, taste-masking agents, surfactants, lipids, antioxidants, and co-solvents, which may be used to enhance or improve solubility, for example ethanol, or a glycol.

In specific embodiments, the medically active liquid as described above may be essentially free of a propellant.

In further specific embodiments, the medically active liquid as described above may be an aqueous solution, in which one or more active pharmaceutical ingredients as described above are dissolved and solubilized in a liquid carrier solution comprising water. Such aqueous solutions optionally may also comprise one or more excipients as described above.

In a further aspect, the invention provides a method for the treatment, stabilization or prevention of a pulmonary disease or condition in a subject by inhalative administration of a medically active liquid in aerosolized form to said subject, wherein the medically active liquid in aerosolized form is generated and administered to said patient by an inhalation device according to the third aspect of the invention comprising a cartridge according to first aspect and a corresponding holder according to the second aspect of the invention.

Furthermore, the present invention relates to the use of an inhalation device according to claim 10 the treatment, stabilization or prevention of a pulmonary disease or condition by inhalative administration of a medically active liquid in aerosolized form to a subject in need thereof, wherein the medically active liquid in aerosolized form is generated and administered to said subject by an inhalation according to the third aspect of the invention comprising a cartridge according to first aspect and a corresponding holder according to the second aspect of the invention.

The subject as referred to above may be an animal, specifically a warm-blooded animal such as a mammal or a human in need of such treatment.

Examples of the pulmonary disease or condition to be treated, stabilized or prevented as referred to above comprise, but are not limited to asthma and chronic obstructive pulmonary disease (COPD).

In yet a further aspect, the invention also relates to a method for providing a pressure compensation to the collapsible inner bag-containing interior of the outer container of a cartridge, specifically to the cartridge of the first aspect of the invention. For the sake of conciseness, reference is made to the definitions above, and in particular, to a cartridge with a cap being deformable, particularly by radial compression forces, preferably by means of at least one protrusion being located at the outer circumference of the cap, and/or at the inner circumference of the holder.

According to this aspect of the invention, initially, before the cartridge is connected to a holder being defined as above as well, the pressure compensation opening is closed, and subsequently, when the cartridge is connected to the holder, an air gap is provided which allows for a pressure equalization of the bag-containing interior with the ambient pressure via the pressure compensation opening. Said air gap may, for example, be formed by way of mechanically deforming (i) either a pressure compensation opening comprising region of the outer container, and/or (ii) a compensation opening covering region of the cap.

In other words, according to one embodiment, the outer container comprises, preferably at the top or downstream end where the cap closes the container, a region or area in which a pressure compensation opening is located as described in further detail above in connection with the cartridge of the first aspect of the invention. When this region is deformed, an air gap between said region and the cap is provided.

According to another embodiment, the cap provides a region which covers aforesaid compensation opening, the latter still being located in the container. By deforming the cap (and not the container), the air gap is provided.

Of course, both alternative embodiments can be combined with each other.

According to specific embodiments, the mechanical deformation is effected upon connecting the cartridge to the holder by exerting a radial compression force from the holder to the cap. In order to avoid repetitions, reference is made to the above explanations. Of course, other ways of effecting a deformation upon exertion of forces to the cap and/or container are possible as well.

According to further specific embodiments, upon subsequent detaching the cartridge from the holder, the mechanical deformation is reversed, such that the air gap is closed and the compensation opening is again covered by the cap, specifically in an airtight manner.

The advantages of the method according to this aspect of the invention are apparent: The pressure compensation opening is only open when the air gap is provided, which in turn is only the case when the cartridge is inserted in the holder. Therefore, when not being used, the cartridge can be detached, the pressure compensation opening closes, and a (then both undesired and unnecessary) exchange of air between the inner of the container and the outside becomes impossible.

### Detailed Description of the Figures

- Fig. 1: shows a schematic cross-sectional view of a cartridge according to the invention in a disconnected state;
- Fig. 2: shows this cartridge in a connected state;
- Fig. 3: shows one embodiment of a cartridge with the cap detached from the container;
- Fig. 4: shows this embodiment with both components attached;
- Fig. 5: shows a cartridge detached from a holder; and
- Fig. 6: shows a cut view of a detail of the cartridge according to Fig. 5.

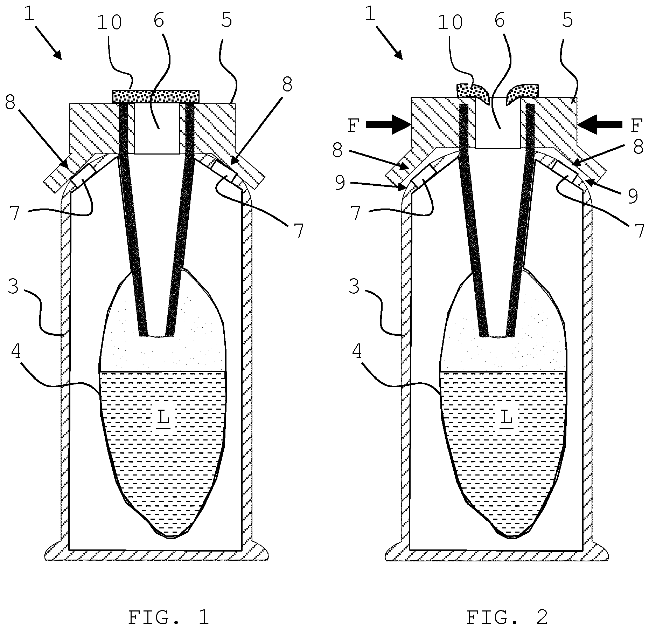

In Fig. 1, a schematic cross-sectional view of a cartridge 1 according to the invention in a disconnected state is shown. This is the situation when the cartridge 1 is detached from the inhalation device.

Cartridge 1 comprises a collapsible inner bag 4 which is enclosed by a container 3. An access opening 6 which is sealed by a lid 10 is located in the top (or downstream) region of the container 3, providing (when opened) access to the interior of bag 4 where medically active liquid L is present.

At the shoulders of container 3, two pressure compensation openings 7 are present. Both pressure compensation openings 7 are located in a region 8 which is (or can be) covered by the cap 5 and are located on opposite sides of the container 3. These openings 7 can provide a connection of the interior of container 3 (but not of bag 4) with the outside. As can be seen in Fig. 1, the cap 5 closes said openings 7 in an airtight manner.

As can be seen in Fig. 2 which depicts the cartridge 1 in a connected state (holder not shown), the cap 5 is deformed by radially acting compression forces F. Also, seal 10 is broken, so that the liquid L could pass access opening 6.

Because of the deformation, there is no longer an airtight closure of pressure compensation openings 7 by means of the cap, and an air gap 9 is provided at each opening 7. Thus, a pressure compensation between interior of container 3 and outside becomes possible. If the volume of bag 4 decreases due to the continuous usage of the device leading to a decrease of the amount of liquid L in the inner bag 4, the resulting pressure difference between the interior and the exterior space of the container 3 can be compensated effectively.

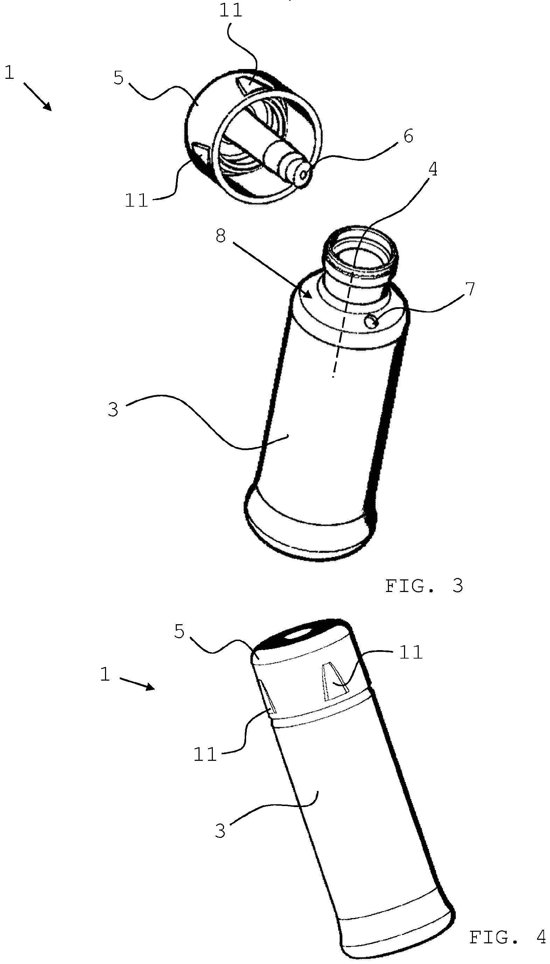

Fig. 3 shows one embodiment of a cartridge 1 with the cap 5 detached from the container 3. The access opening 6 is designed as a long hollow tube which can reach into the barely visible collapsible bag 4, when the cap 5 is attached to the container 3.

Protrusions 11 are present on the circumference of the outside of cap 5. If forces act onto these protrusions 11, cap 5 deforms in a way such that an air gap (not visible) is provided which allows a fluid connection of the inside of container 3 and the outside through pressure compensation openings 7. This deformation is reversible, such that, when the force is removed, the openings 7 are closed again. In order to allow closure of said openings 7 by cap 5, they are arranged in a region 8 which is (or can be) covered by cap 5.

As can be seen in Fig. 3 as well, cap 5 is fixed to container 3 by way of a clip closure. The respective geometrical features are present at the top end of container 3 as well as inside the top end of cap 5. If a suitable material is chosen for cap 5 which preferably is elastically deformable, no additional seal or the like is necessary in order to provide an air- and liquid tight closure, neither for the interface of cap 5 and container 3, as well as inside of cap 5 and aforesaid region 8 where the pressure compensation opening 7 are located. This results in a simple setup, requiring a minimal number of components.

The attached components of Fig. 3 can be seen in Fig. 4. The assembled cartridge 1 can easily be inserted into a holder (not shown), but also shelved for later use. When detached from a holder, pressure compensation opening(s) (not visible) are closed in order to interrupt any gas exchange between inside of the container 3 and outside.

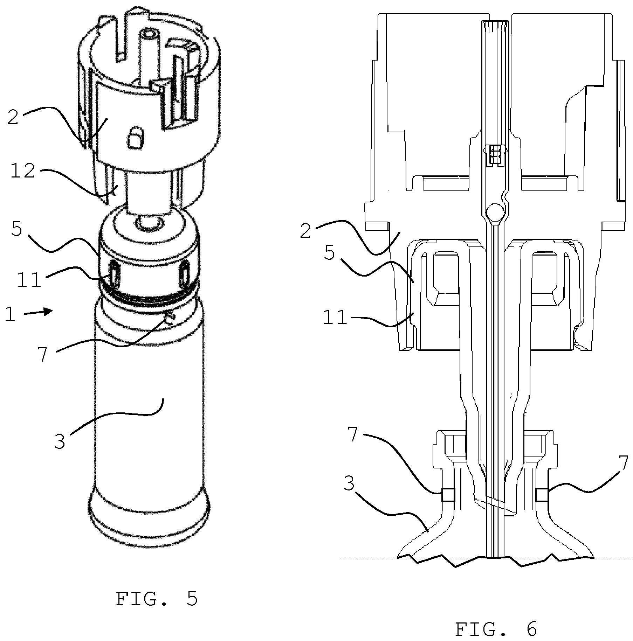

In Fig. 5, a cartridge 1 detached from a holder 2 is depicted. As can be seen, holder 2 is designed to clamp cartridge 1 by cap 5. Protrusions 11 fit in corresponding recesses 12 which are designed to inhibit rotation of the inserted cartridge 1 and exert compressive forces (not shown) onto said protrusions 11 in order to provide an air gap (not shown) when holder 2 holds cartridge 1. Opening 7 is located in the "neck" region of the cartridge; however, it can also be located in the "shoulder" region as depicted in Fig. 3.

Fig. 6 shows a detailed sectional view of the cartridge 1 and holder 2. In this drawing, cap 5 is attached to holder 2. Forces (not shown) exert pressure onto the protrusions 11 (one shown). By deformation of cap 5, its inside surface is partially spaced apart from the openings 7 such that an air gap (not shown) is provided for pressure compensation into the container 3 (bag 4 not shown).

### List of reference signs:

- 1: cartridge
- 2: holder
- 3: container
- 4: collapsible inner bag
- 5: cap
- 6: access opening
- 7: pressure compensation opening
- 8: region
- 9: air gap
- 10: lid
- 11: protrusion
- 12: recess
- L: liquid
- F: force

## Claims

1. Cartridge (1) for the storage of a medically active liquid (L) and for supply of the same to a pumping unit of an inhalation device,
- said inhalation device comprising a housing, a pumping unit which can be fluidically connected with said cartridge (1), a holder (2) for said cartridge (1), and a nozzle which is fluidically connected downstream of said pumping unit,
- wherein, when the pumping unit is fluidically connected to the cartridge (1), the pumping unit is capable of generating a negative gauge pressure such as to draw medically active liquid (L) from the cartridge (1) into the pumping unit, and of generating a positive gauge pressure such as to press said liquid (L) from said pumping unit into and through the nozzle such as to atomize said liquid (L),
- wherein the cartridge (1) comprises an outer container (3) enclosing a collapsible inner bag (4), and a cap (5) which is capable of closing the container (3), the cap (5) further providing an openable access opening (6) to the inner bag (4),
- wherein further, the container (3) of the cartridge (1) has at least one pressure compensation opening (7),
and wherein said at least one pressure compensation opening (7) is located in a region (8) of the container (3) which is covered by the cap (5).

2. Cartridge (1) according to claim 1, wherein, in a state when the cartridge (1) is disconnected from the holder (2), the at least one pressure compensation opening (7) is covered by the cap (5), while in a state when the cartridge (1) is connected to the holder (2), said pressure compensation opening (7) is open.

3. Cartridge according to claim 2, wherein, in a state when the cartridge (1) is disconnected from the holder (2), the at least one pressure compensation opening (7) is covered airtight by the cap (5).

4. Cartridge (1) according to any one of claims 1 to 3, wherein the cap (5) and/or at least a cap connection region of the outer container (3) are made of a deformable material, and can be deformed such that an air gap (9) is provided between the inside surface of the cap (5) and the outside surface of the outer container which faces the cap (3).

5. Cartridge (1) according to claim 4, wherein the cap (5) is deformable by a radial compression force (F).

6. Cartridge (1) according to claim 4 or 5, wherein at the outer circumference of the cap (5), and/or at the inner circumference of the holder (2), at least one protrusion (11) is present, which, in the connected state of the cartridge (1), causes the cap (5) to be deformed such as to provide said air gap (9), preferably wherein the cap (5) and/or the holder (2) have a plurality of evenly spaced protrusions (11).

7. Cartridge according to any one of claims 1 to 6, wherein the container (3) of the cartridge (1) has a plurality of pressure compensation openings (7), preferably wherein the container (3) of the cartridge (1) has two pressure compensation openings (7).

8. Holder (2) connected to a cartridge (1) as defined in any of the preceding claims, wherein the holder (2) is adapted to releasably connect to the cartridge (1) by clamping its cap (5).

9. Holder (2) according to claim 8, wherein, by way of a connection of the holder (2) with a cartridge (1) as defined in claim 4 to 7, the air gap (9) can be provided.

10. Holder (2) according to claim 9, wherein, when the cap (5) is connected to the holder (2), the holder (2) is configured to exert a compression force (F) onto the cap (5).

11. Inhalation device for delivering a medically active liquid in the form of an aerosol, said inhalation device being adapted for holding or receiving a cartridge according to any one of claims 1 to 7 and comprising a holder according to any one of claims 8 to 10 for holding said cartridge.

12. Inhalation device for delivering a medically active liquid in the form of an aerosol according to claim 11, said inhalation device comprising a cartridge (1) according to any one of claims 1 to 7 and a holder (2) according to any one of claims 8 to 10 for holding said cartridge.

13. Method for providing a pressure compensation to the collapsible inner bag-containing interior of the outer container (3) of a cartridge (1) as defined in any of claims 4 to 7, wherein initially, before the cartridge (1) is connected to a holder (2) as defined in any of claims 8 to 10, the pressure compensation opening (7) is closed, and wherein subsequently, when the cartridge (1) is connected to the holder (2), by way of mechanically deforming (i) a pressure compensation opening comprising region of the outer container (3) and/or (ii) a compensation opening covering region of the cap (5), an air gap (9) is provided which allows for a pressure equalization of the bag-containing interior with the ambient pressure, preferably wherein the mechanical deformation is effected upon connecting the cartridge (1) to the holder (2) by exerting a radial compression force (F) from the holder (2) to the cap (5).

14. Method according to claim 13, wherein, upon subsequent detaching the cartridge (1) from the holder (2), the mechanical deformation is reversed, such that the air gap (9) is closed and the compensation opening (7) is again airtight covered by the cap (5).

## Patentansprüche

1. Kartusche (1) für die Lagerung einer medizinisch wirksamen Flüssigkeit (L) und für die Zuführung derselben zu eine Pumpeinheit einer Inhalationsvorrichtung,
- wobei die Inhalationsvorrichtung ein Gehäuse, eine Pumpeinheit, die fluidisch mit der Kartusche (1) verbunden werden kann, einen Halter (2) für die Kartusche (1) und eine Düse umfasst, die stromabwärts von der Pumpeinheit fluidisch verbunden ist,
- wobei die Pumpeinheit in der Lage ist, wenn die Pumpeinheit fluidisch mit der Kartusche (1) verbunden ist, einen Unterdruck zu erzeugen, um medizinisch wirksame Flüssigkeit (L) aus der Kartusche (1) in die Pumpeinheit zu saugen, und einen Überdruck zu erzeugen, um die Flüssigkeit (L) aus der Pumpeinheit in und durch die Düse zu drücken, um die Flüssigkeit (L) zu zerstäuben,
- wobei die Kartusche (1) einen äußeren Behälter (3), der einen zusammenfaltbaren inneren Beutel (4) umschließt, und eine Kappe (5) umfasst, die den Behälter (3) verschließen kann, wobei die Kappe (5) ferner eine Zugangsöffnung (6) zu dem inneren Beutel (4) bereitstellt, die sich öffnen lässt,
- wobei ferner der Behälter (3) der Kartusche (1) mindestens eine Druckausgleichsöffnung (7) hat,
und wobei die Druckausgleichsöffnung (7) in einem Bereich (8) des Behälters (3) angeordnet ist, der von der Kappe (5) abgedeckt wird.

2. Kartusche (1) nach Anspruch 1, wobei die mindestens eine Druckausgleichsöffnung (7) in einem Zustand, wenn die Kartusche (1) von dem Halter (2) getrennt ist, von der Kappe (5) abgedeckt ist, während die Druckausgleichsöffnung (7) in einem Zustand, wenn die Kartusche (1) mit dem Halter (2) verbunden ist, offen ist.

3. Kartusche (1) nach Anspruch 2, wobei die mindestens eine Druckausgleichsöffnung (7) in einem Zustand, wenn die Kartusche (1) von dem Halter (2) getrennt ist, luftdicht von der Kappe (5) abgedeckt ist.

4. Kartusche (1) nach einem der Ansprüche 1 bis 3, wobei die Kappe (5) und/oder mindestens ein Kappenverbindungsbereich des äußeren Behälters (3) aus einem deformierbaren Material hergestellt sind und deformiert werden können, so dass ein Luftspalt (9) zwischen der Innenfläche der Kappe (5) und der Außenfläche des äußeren Behälters, die der Kappe (3) zugewandt ist, vorgesehen ist.

5. Kartusche (1) nach Anspruch 4, wobei die Kappe (5) durch eine radiale Druckkraft (F) deformierbar ist.

6. Kartusche (1) nach Anspruch 4 oder 5, wobei an dem äußeren Umfang der Kappe (5) und/oder an dem inneren Umfang des Halters (2) mindestens ein Vorsprung (11) vorliegt, der in dem verbundenen Zustand der Kartusche (1) veranlasst, dass die Kappe (5) deformiert wird, um den Luftspalt (9) bereitzustellen, vorzugsweise wobei die Kappe (5) und/oder der Halter (2) eine Vielzahl von gleichmäßig beabstandeten Vorsprüngen (11) haben.

7. Kartusche nach einem der Ansprüche 1 bis 6, wobei der Behälter (3) der Kartusche (1) eine Vielzahl von Druckausgleichsöffnungen (7) hat, vorzugsweise wobei der Behälter (3) der Kartusche (1) zwei Druckausgleichsöffnungen (7) hat.

8. Halter (2), der mit einer Kartusche (1) verbunden ist, wie sie in einem der vorhergehenden Ansprüche definiert wird, wobei der Halter (2) dazu ausgeführt ist, mit der Kartusche (1) freigebbar verbunden zu werden, indem er ihre Kappe (5) einklemmt.

9. Halter (2) nach Anspruch 8, wobei der Luftspalt (9) mittels einer Verbindung des Halters (2) mit einer Kartusche (1), wie in Ansprüchen 4 bis 7 definiert, bereitgestellt werden kann.

10. Halter (2) nach Anspruch 9, wobei der Halter (2) dazu ausgestaltet ist, eine Druckkraft (F) auf die Kappe (5) auszuüben, wenn die Kappe (5) mit dem Halter (2) verbunden ist.

11. Inhalationsvorrichtung zum Zuführen einer medizinisch wirksamen Flüssigkeit in der Form eines Aerosols, wobei die Inhalationsvorrichtung dazu ausgeführt ist, eine Kartusche nach einem der Ansprüche 1 bis 7 zu halten oder aufzunehmen, und einen Halter nach einem der Ansprüche 8 bis 10 zum Halten der Kartusche umfasst.

12. Inhalationsvorrichtung zum Zuführen einer medizinisch wirksamen Flüssigkeit in der Form eines Aerosols nach Anspruch 11, wobei die Inhalationsvorrichtung eine Kartusche (1) nach einem der Ansprüche 1 bis 7 und einen Halter (2) nach einem der Ansprüche 8 bis 10 zum Halten der Kartusche umfasst.

13. Verfahren zur Bereitstellung von Druckausgleich für das den zusammenfaltbaren inneren Beutel enthaltende Innere des äußeren Behälters (3) einer Kartusche (1), wie in einem der Ansprüche 4 bis 7 definiert, wobei anfänglich, bevor die Kartusche (1) mit einem Halter (2), wie in einem der Ansprüche 8 bis 10 definiert, verbunden ist, die Druckausgleichsöffnung (7) geschlossen ist, und wobei nachfolgend, wenn die Kartusche (1) mit dem Halter (2) verbunden ist, durch mechanisches Deformieren (i) eines eine Druckausgleichsöffnung umfassenden Bereichs des äußeren Behälters (3) und/oder (ii) eines eine Ausgleichsöffnung abdeckenden Bereichs der Kappe (5) ein Luftspalt (9) bereitgestellt wird, der einen Druckausgleich des den Beutel enthaltenden Inneren mit dem Umgebungsdruck gestattet, vorzugsweise wobei die mechanische Deformierung bei Verbinden der Kartusche (1) mit dem Halter (2) durchgeführt wird, indem eine radiale Druckkraft (F) von dem Halter (2) auf die Kappe (5) ausgeübt wird.

14. Verfahren nach Anspruch 13, wobei die mechanische Deformierung bei nachfolgendem Ablösen der Kartusche (1) von dem Halter (2) umgekehrt wird, so dass der Luftspalt (9) geschlossen wird und die Ausgleichsöffnung (7) wieder luftdicht von der Kappe (5) abgedeckt ist.

## Revendications

1. Cartouche (1) destinée à stocker un liquide médicalement actif (L) et à le fournir à une unité de pompage d'un dispositif d'inhalation,
- ledit dispositif d'inhalation comprenant un boîtier, une unité de pompage qui peut être raccordée fluidiquement à ladite cartouche (1), un support (2) destiné à ladite cartouche (1), et une buse qui est raccordée fluidiquement en aval de ladite unité de pompage,
- lorsque l'unité de pompage est raccordée fluidiquement à la cartouche (1), l'unité de pompage étant capable de générer une pression manométrique négative de manière à aspirer un liquide médicalement actif (L) de la cartouche (1) jusque dans l'unité de pompage, et de générer une pression manométrique positive de manière à presser ledit liquide (L) de ladite unité de pompage jusque dans la buse et à travers celle-ci de manière à atomiser ledit liquide (L),
- la cartouche (1) comprenant un conteneur extérieur (3) renfermant un sac intérieur souple (4), et un capuchon (5) qui est capable de fermer le conteneur (3), le capuchon (5) fournissant en outre une ouverture d'accès (6) pouvant s'ouvrir sur le sac intérieur (4),
- en outre, le conteneur (3) de la cartouche (1) comportant au moins une ouverture de compensation de pression (7),
et ladite au moins une ouverture de compensation de pression (7) étant située dans une région (8) du conteneur (3) qui est recouverte par le capuchon (5).

2. Cartouche (1) selon la revendication 1, dans un état dans lequel la cartouche (1) est retirée du support (2), l'au moins une ouverture de compensation de pression (7) étant recouverte par le capuchon (5), tandis que dans un état dans lequel la cartouche (1) est raccordé au support (2), ladite ouverture de compensation de pression (7) étant ouverte.

3. Cartouche selon la revendication 2, dans un état dans lequel la cartouche (1) est retirée du support (2), l'au moins une ouverture de compensation de pression (7) étant recouverte par le capuchon (5) de manière étanche à l'air.

4. Cartouche (1) selon l'une quelconque des revendications 1 à 3, le capuchon (5) et/ou au moins une région de raccordement de capuchon du conteneur extérieur (3) étant en une matière déformable et pouvant être déformés de manière à ménager un espace d'air (9) entre la surface intérieure du capuchon (5) et la surface extérieure du conteneur extérieur qui fait face au capuchon (3).

5. Cartouche (1) selon la revendication 4, le capuchon (5) étant déformable par une force de compression radiale (F).

6. Cartouche (1) selon la revendication 4 ou 5, au moins une saillie (11) étant présente au niveau de la circonférence extérieure du capuchon (5), et/ou au niveau de la circonférence intérieure du support (2), laquelle saillie provoque, dans l'état dans lequel la cartouche (1) est raccordée, la déformation du capuchon (5) de manière à ménager ledit espace d'air (9), de préférence le capuchon (5) et/ou le support (2) comportant une pluralité de saillies (11) régulièrement espacées.

7. Cartouche selon l'une quelconque des revendications 1 à 6, le conteneur (3) de la cartouche (1) comportant une pluralité d'ouvertures de compensation de pression (7), de préférence le conteneur (3) de la cartouche (1) comportant deux ouvertures de compensation de pression (7).

8. Support (2) raccordé à une cartouche (1) telle que définie dans l'une quelconque des revendications précédentes, le support (2) étant adapté pour être raccordé de manière amovible à la cartouche (1) en serrant son capuchon (5).

9. Support (2) selon la revendication 8, un raccordement du support (2) à une cartouche (1) telle que définie dans les revendications 4 à 7, permettant de ménager l'espace d'air (9).

10. Support (2) selon la revendication 9, lorsque le capuchon (5) est raccordé au support (2), le support (2) étant conçu pour exercer une force de compression (F) sur le capuchon (5).

11. Dispositif d'inhalation destiné à délivrer un liquide médicalement actif sous forme d'aérosol, ledit dispositif d'inhalation étant adapté pour supporter ou recevoir une cartouche selon l'une quelconque des revendications 1 à 7 et comprenant un support selon l'une quelconque des revendications 8 à 10 destiné à supporter ladite cartouche.

12. Dispositif d'inhalation destiné à délivrer un liquide médicalement actif sous forme d'aérosol selon la revendication 11, ledit dispositif d'inhalation comprenant une cartouche (1) selon l'une quelconque des revendications 1 à 7 et un support (2) selon l'une quelconque des revendications 8 à 10 destiné à supporter ladite cartouche.

13. Procédé de fourniture d'une compensation de pression à l'intérieur, contenant un sac intérieur souple, du conteneur extérieur (3) d'une cartouche (1) telle que définie dans l'une quelconque des revendications 4 à 7, l'ouverture de compensation de pression (7) étant fermée initialement avant que la cartouche (1) ne soit raccordée à un support (2) tel que défini dans l'une quelconque des revendications 8 à 10, puis, lorsque la cartouche (1) est raccordée au support (2), une déformation mécanique (i) d'une ouverture de compensation de pression comprenant une région du conteneur extérieur (3) et/ou (ii) d'une ouverture de compensation recouvrant une région du capuchon (5) permettant de ménager un espace d'air (9) qui permet d'égaliser la pression à l'intérieur, contenant un sac, à la pression ambiante, de préférence la déformation mécanique étant effectuée lors du raccordement de la cartouche (1) au support (2) par une force de compression radiale (F) exercée par le support (2) sur le capuchon (5).

14. Procédé selon la revendication 13, lorsque la cartouche (1) est par la suite détachée du support (2), la déformation mécanique étant inversée de façon à fermer l'espace d'air (9) et de manière à ce que l'ouverture de compensation (7) soit à nouveau hermétiquement recouverte par le capuchon (5).
